# EUROPEAN PATENT APPLICATION

(11) **EP 4 603 251 A1**
(43) Date of publication of application: **20.08.2025**
(21) Application number: 24158131.3
(22) Date of filing: 16.02.2024
(51) Int. Cl.: B29B 17/00, G01N 33/44, B29C 48/00, B29C 48/05, B29K 23/00, B29K 105/00, B29K 105/26

(54) **A METHOD FOR DETERMINING RE-UTILIZATION EFFICIENCY OF ADDITIVES IN POST CONSUMER RECYCLED BLENDS**

(71) Applicant: Basell Polyolefine GmbH, 50389 Wesseling (DE)
(72) Inventor: RIEMMA, Antonio, 44122 Ferrara (IT); ELSAS, Katharina, 65926 Frankfurt/M. (DE); MANNEBACH, Gerd, 65926 Frankfurt/M. (DE)
(74) Representative: LyondellBasell

(57) **Abstract**

The present application provides a method for determining re-utilization efficiency of an additive content in Post-Consumer Recycled (PCR) blends. The disclosed method enables that the quantified additive content, enables end user to correlate the efficiency of the additive content in PCR with number of times of re-utilization of the PCR blend. The present application also provides a method for producing a molded article with optimized additive content. The molded article produced thereof resulted in longevity and effectiveness of additives in PCR blends enhancing performance properties of the article.

## Description

### FIELD OF THE DISCLOSURE

The present disclosure generally relates to a method for determining efficiency of additives in Post-Consumer Recycled (PCR) blends. Specifically, the disclosure relates to a method for determining re-utilization efficiency of the additives in PCR blends, and also a method of producing a molded article thereof. More particularly, the disclosed method enables end user to correlate the efficiency of the additive content in PCR with number of times of re-utilization of the PCR blend.

### BACKGROUND OF THE DISCLOSURE

Managing the lifecycle of polymer-based materials is crucial for maintaining equilibrium in a circular economy, especially in products that incorporate plastics. Given that plastics are often derived from petroleum sources and typically known to be non-biodegradable, there is a widespread recognition of the imperative to establish sustainable and efficient post-industrial polymer recycling processes across various industries on a global scale. Developing a solution for multiple-processing and recycling Post-Industrial and Post-Consumer Recycled (PCR) polymers is essential for fostering a forward-looking approach in industries and addressing both environmental and economic concerns. Despite the substantial demand for post-industrial and post-consumer polymer materials, there is ongoing need and demand for ensuring the end molded products high quality.

It is well-known that plastic waste particularly coming from post-consumer plastic items, presents a significant environmental challenge due to its non-biodegradable nature. To address such significant threat to the environment several recycling methods have been developed, to convert plastic waste into recycled polymers, i.e., PCR polymers. However, the repeated re-utilization of these recycled polymers poses challenges in maintaining the performance and stability of additives introduced during their initial production or compounding process.

Additives, such as antioxidants, stabilizers, and colorants, play a crucial role in enhancing the properties of polymers. Traditional methods for analyzing additives in recycled polymers have often been limited in their ability to provide a thorough understanding of the additive behavior over multiple recycling cycles.

Existing analytical methods or models lack in the specificity in evaluating how additives evolve or degrade during re-utilization, hindering the efforts to optimize recycling processes or assess optimised additive content to ensure the quality of recycled materials. Consequently, there is a need for technically advanced analytical approach or methodology that enables the end user to arrive at a precise quantification of the additive content and facilitates a comprehensive understanding of additive behavior during PCR blend re-utilization.

In view of the foregoing, the inventors of the present disclosure have significantly advanced the state of the art in the field of polymer recycling by offering a methodology for determining the additive content in PCR blends, thereby contributing to the sustainable utilization, followed by re-utilization of plastic waste and the production of high-quality recycled materials.

To this end, an object of the present disclosure is to provide a method for determining re-utilization efficiency of additives in Post-Consumer Recycled (PCR) blends which enables the end user to correlate the efficiency of quantified additive content in the PCR with number of times of re-utilization of the PCR blend.

Another object of the present disclosure is to provide a method for determining additive content in Post-Consumer Recycled (PCR) blends, which enables the end user to quantify the additive content post each re-utilization of the PCR blend, by providing valuable insights into stability, performance and behaviour of additives in PCR blends.

Yet another object of the disclosure is to provide a method for producing a molded article and an article thereof, which contains an optimised amount of additive content in the recycled polymer, exhibits enhanced performance properties for ex., longevity and effectiveness of additives in PCR blends.

### BRIEF SUMMARY OF THE DISCLOSURE

The present disclosure addresses the limitations of existing methods by providing improved method for determining additive content and re-utilization efficiency of the additives in PCR blends.

In one aspect the present disclosure, a method for determining re-utilization efficiency of an additive content in Post-Consumer Recycled (PCR) blends is provided. The method comprises the steps of: a. compounding an obtained Post-Consumer Recycled polymers in a twin-screw extruder, under controlled temperature and pressure conditions; b. collecting samples of the compounded PCR blend at predetermined intervals during the extrusion process; c. quantifying concentrations of individual additives and/or additives degradation products present in the collected PCR blend samples using analytical techniques, thereby determining the additive content and/or additive degradation product content post-utilization; d. subjecting portions of the compounded PCR blend to testing methods to evaluate the efficacy of additives in resisting oxidation after utilization; e. assessing stability and efficiency of additives in recycled materials after utilization; and f. assessing cumulative effect of utilization on the stability and performance of additives in the PCR blends. The quantified additive content thus analyzed, enables end user to correlate the efficiency of the additive content in PCR with number of times of re-utilization of the PCR blend.

In another aspect of the present disclosure, a method for determining additive content in Post-Consumer Recycled (PCR) blends is disclosed. The method comprises the steps of: a. compounding an obtained Post-Consumer Recycled polymers in a twin-screw extruder, under controlled temperature and pressure conditions; b. collecting samples of the compounded PCR blend at predetermined intervals during the extrusion process; c. quantifying concentrations of individual additives present in the collected PCR blend samples using analytical techniques, thereby determining the additive content post-utilization; and d. subjecting portions of the compounded PCR blend to testing methods to evaluate the efficacy of additives in resisting oxidation after utilization.

In yet another aspect, a method for producing a molded article and a molded article thereof is also disclosed. The method comprises the steps of: a) providing a recycled polymer feedstock; b) extruding the recycled polymer feedstock at a temperature of 170 to 240°C; c) obtaining recycled polymer pellets; d) blow molding the recycled polymer pellets to form a recycled polymer product; e) adding the additive in step (a) determined by correlating the efficiency of the additive content in PCR with number of times of re-utilization of the PCR blend. The addition of optimized content of the additive to the recycled polymer results in a molded article exhibiting longevity and effectiveness of additives in PCR blends.

The foregoing has outlined rather broadly the features and technical advantages of the present disclosure in order that the detailed description of the disclosure that follows may be better understood. Additional features and advantages of the disclosure will be described hereinafter, which form the subject matter of the claims of the disclosure. It should be appreciated by those skilled in the art that the conception and specific embodiments disclosed may be readily utilized as a basis for modifying or designing molded containers or articles (injection, compression, or blow molded; thermoformed; or extruded), and/or processes for carrying out the same purposes of the present disclosure. It should also be realized by those skilled in the art that such equivalent constructions do not depart from the spirit and scope of the disclosure as set forth in the appended claims. The novel features which are believed to be characteristic of the disclosure, both as to its structure and method of manufacture, together with further objects and advantages will be better understood from the following description.

### BRIEF DESCRIPTION OF THE DRAWINGS

Embodiments are illustrated by way of example and are not limited in the accompanying figures.
FIG. 1 illustrates a flow chart of a method for determining re-utilization efficiency of an additive content in Post-Consumer Recycled (PCR) blends, in accordance with one embodiment of the present disclosure.
FIG. 2 illustrates a flow chart of a method for determining additive content in Post-Consumer Recycled (PCR) blends, in accordance with one embodiment of the present disclosure.

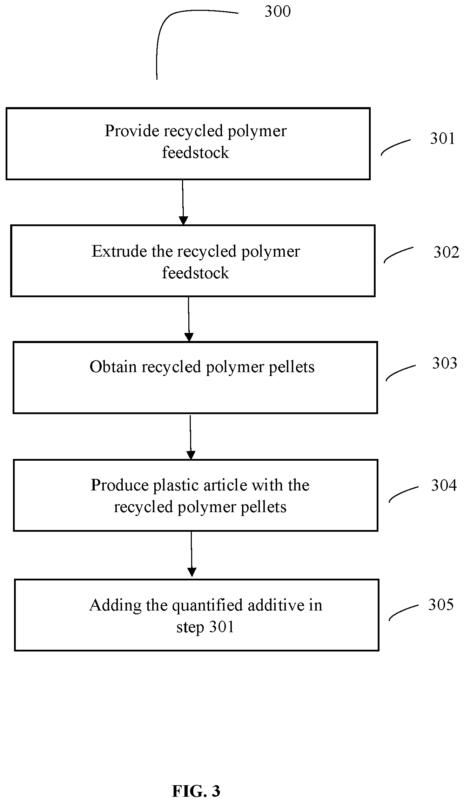

FIG. 3 illustrates a flow chart of a method for producing a molded article, in accordance with one embodiment of the present disclosure.

### DETAILED DESCRIPTION OF THE DISCLOSURE

Illustrative embodiments of the subject matter claimed below will now be disclosed. In the interest of clarity, some features of some actual implementations may not be described in this specification. It will be appreciated that in the development of any such actual embodiments, numerous implementation-specific decisions must be made to achieve the developer's specific goals, such as compliance with system-related and business-related constraints, which will vary from one implementation to another. Moreover, it will be appreciated that such a development effort, even if complex and time-consuming, would be a routine undertaking for those of ordinary skill in the art having the benefit of this disclosure.

The words and phrases used herein should be understood and interpreted to have a meaning consistent with the understanding of those words and phrases by those skilled in the relevant art. No special definition of a term or phrase, i.e., a definition that is different from the ordinary and customary meaning as understood by those skilled in the art, is intended to be implied by consistent usage of the term or phrase herein. To the extent that a term or phrase is intended to have a special meaning, i.e., a meaning other than the broadest meaning understood by skilled artisans, such a special or clarifying definition will be expressly set forth in the specification in a definitional manner that provides the special or clarifying definition for the term or phrase. It must also be noted that, as used in the specification and the appended claims, the singular forms "a," "an," and "the" include plural references unless otherwise specified.

For example, the following discussion contains a non-exhaustive list of definitions of several specific terms used in this disclosure (other terms may be defined or clarified in a definitional manner elsewhere herein). These definitions are intended to clarify the meanings of the terms used herein. It is believed that the terms are used in a manner consistent with their ordinary meaning, but the definitions are nonetheless specified here for clarity.

As used in this specification and the claims, the terms "comprising," "containing," or "including" mean that at least the named compound, element, material, particle, or method step is present in the composition, the article, or the method, but does not exclude the presence of other compounds, elements, materials, particles, or method steps even if the other such compounds, elements, materials, particles, or method steps have the same function as that which is named, unless expressly excluded in the claims. It is also to be understood that the mention of one or more method steps does not preclude the presence of additional method steps before or after the combined recited steps or intervening method steps between those steps expressly identified.

Moreover, it is also to be understood that the lettering of process steps or ingredients is for identifying discrete activities or ingredients and the recited lettering can be arranged in any sequence, unless expressly indicated.

For the purpose of the present description and of the claims which follow, except where otherwise indicated, numbers expressing amounts, quantities, percentages, and so forth, are to be understood as being modified by the term "about". Also, ranges include any combination of the maximum and minimum points disclosed and include any intermediate ranges therein, which may or may not be specifically enumerated herein.

Wherever possible, the same reference numbers will be used throughout the drawingsto refer to the same or the like parts. Embodiments disclosed herein are related to a method of determining the re-utilization efficiency of an additive content in Post-Consumer Recycled (PCR) b l end s, and a molded article produced thereof by employing the methods according to the teachings of the present disclosure.

As used herein the phrase "Recycled Polymers" or "Post-Consumer Recycled" (PCR) can be used interchangeably, refers to materials sourced from products that have been previously used. These materials originate from industrial waste, with PCR materials specifically derived from end-user waste that has undergone collection and recycling.

As used herein, the term "post-consumer" refers to a source of material that originates after the end consumer has used the material in a consumer good or product.

As used herein the term "additive", refers to a diverse range of substances introduced during the recycling process to enhance the performance, properties, or appearance of the recycled material. These additives in accordance with the present disclosure may include stabilizers, colorants, reinforcing agents, and processing aids, among others, aiming to address potential degradation during recycling and improve the overall quality of the final recycled product.

As used herein the phrase "additive degradation", refers to additives behaviour, efficiency, stability and performance properties being affected by the utilization and re-utilization of the additve in PCR blends.

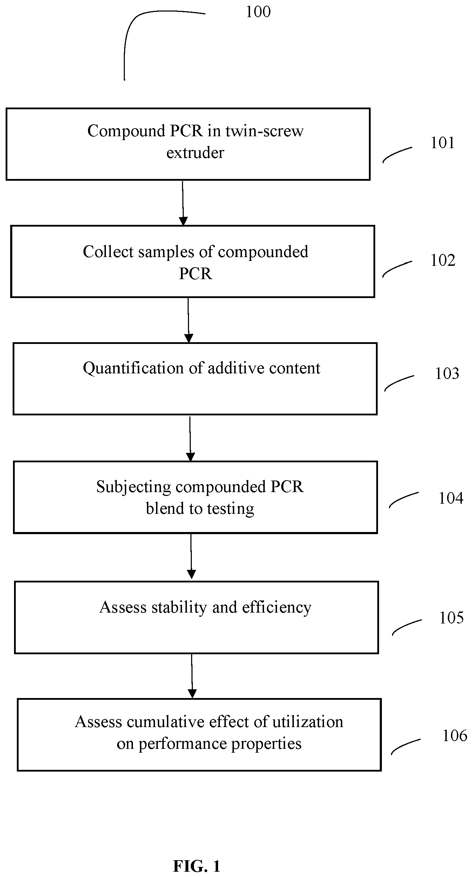

FIG. 1 illustrates a flow chart 100 of a method for determining re-utilization efficiency of an additive content in Post-Consumer Recycled (PCR) blends in accordance with the present disclosure. As shown in FIG. 1, the method involves step 101, compounding an obtained PCR polymers in a twin-screw extruder. In accordance wiyh the present disclosure the PCR polymer compounded in the twin-screw extruder is obtained from plastic waste sources including but not limited to recycled polypropylene, recycled polyethylene, preferably recycled high density polyethylene(HDPE), recycled low density polyethylene(LDPE), recycled linear low density polyethylene(LLDPE), recycled medium density polyethylene(MDPE), or a mixture thereof. In a preferred embodiment, the PCR polymer compounded in the twin-screw extruder is recycled polyethylene or polypropylene. In a most preferred embodiment, the PCR polymer compounded in the twin-screw extruder is recycled polyethylene.

In another embodiment, the obtained polymer is recycled polystyrene. In yet another embodiment, the polymer is poly(dimethylsiloxane). In another embodiment, the obtained polymer is a polypropylene homopolymer or a primarily polypropylene copolymer. In another embodiment, the obtained polymer is a polyethylene homopolymer or a primarily polyethylene copolymer.

The PCR polymer is subjected to compounding in the twin-screw extruder under controlled temperature and pressure conditions. For example at temperatures greater than 180 °C, preferably ranging between 190 °C to 250 °C. In some embodiments of the present disclosure, the PCR polymer is also compounded as a mixture including compatibilizers, pigments, dyes, process aides, additives, and/or a mixture thereof in the twin-screw extruder under controller temperature and pressure to facilitate homogeneous mixing and distribution of components.

Non-limiting examples of pigments include organic pigments, such as copper phthalocyanine, inorganic pigments, such as titanium dioxide, and other pigments that may be apparent to those having ordinary skill in the art. A non-limiting example of an organic dye includes Basic Yellow 51. Non-limiting examples of process aides are antistatic agents, such as glycerol monostearate and slip-promoting agents, such as erucamide. Suitable compatibilizers include for example, but not limited to maleic anhydride grafted polymers, polyethylene-grafted maleic anhydride, polypropylene-grafted maleic anhydride, silane grafted polyethylene, acrylic acid grafted polyethylene, glycidyl acrylate grafted polyethylene, glycidyl methacrylate grafted polypropylene, hydroxyethyl methacrylate grafted polyethylene and aminoethyl methacrylate grafted polypropylene.

The method further involves step 102, where the samples of the compounded PCR blend are collected at predetermined intervals during the extrusion process. The intervals may be defined based on specific time durations or the completion of at least 2, preferably between 5 and 10, extruder revolutions, ensuring a comprehensive representation of the blends composition.

In accordance with the present disclosure, the method 100 involves step 103, quantifying concentrations of individual additives and/or additives degradation products present in the collected PCR blend samples using analytical techniques. The quantification of the concentration of the additives and/or additives degradation products is performed using well known analytical techniques such as High-Performance Liquid Chromatography (HPLC), Gel Permeation Chromatography (GPC), Nuclear Magnetic Resonance (NMR) Spectroscopy, Infrared Spectroscopy (IR), or Mass Spectroscopy (MS). In a preferred embodiment in accordance with the present disclosure, the analytical technique is High-Performance Liquid Chromatography (HPLC).

In accordance with an embodiment of the present disclosure, collected samples in step 102 undergo HPLC analysis 103 to quantify the concentrations of individual additives and/or additives degradation products present in the PCR blend. The HPLC technique aids in separating and quantifying individual additives and/or additives degradation products within the PCR blend, ensuring precise measurement of additive concentrations. The HPLC analysis includes a separation step that isolates individual additives and/or additives degradation products present in the PCR blend, thereby enabling an accurate quantification. The HPLC analysis is conducted using calibration standards, internal standards, or both, to ensure accuracy and reproducibility of additive concentration measurements. The calibration and standardization processes play a critical role in maintaining the reliability of additive concentration measurements.

In an embodiment of the present dislclosure the HPLC analysis 103 results identifying the individual additives content and/or additives degradation products, within the PCR blend is coupled with data analytics techniques, statistical analysis, or machine learning algorithms to extract patterns, trends, and/or correlations which further help in assessing the additive behaviour during re-utilization cycles.

In a further detailed embodiment, in accordance with the present disclosure the HPLC analysis step 103 is seamlessly integrated with advanced data analytics techniques. This may include statistical analysis or machine learning algorithms. This integration allows for the extraction of intricate patterns, trends, and correlations in the behavior of additives during re-utilization cycles.

Additives are common in the art. Types of additives for preparing polyethylene compositions are, for example, antioxidants, melt stabilizers, light stabilizers, acid scavengers, lubricants, processing aids, antiblocking agents, slip agents, antistatic agents, antifogging agents, pigments or dyes, nucleating agents, flame retardants or fillers. It is common that several additives are added. The multiple additives can be different types of additives. It is however also possible that several representatives of one type of additives are added to the low density polyethylene. Additives of all these types are generally commercially available and are described, for example, in Hans Zweifel, Plastics Additives Handbook, *5th* Edition, Munich, 2001.

Non-limiting examples of additives include antioxidants, processing stabilizers, or colorants. In a specific embodiment the additive is selected from the group comprising antioxidants, stabilizers, or colorants, including BHT (Butylated Hydroxytoluene), BHA (Butylated Hydroxyanisole), Tris(2,4-di-tert-butylphenyl)phosphite (TNPP), Triphenyl phosphite, Dilauryl thiodipropionate (DLTDP), Dimyristyl thiodipropionate (DMTDP), Calcium stearate, Zinc stearate, Titanium dioxide, Iron oxide pigments, Fatty acid esters and / or Calcium stearate. In a preferred embodiment the additive is selected from the group comprising antioxidants, stabilizers, Calcium stearate, Zinc stearate.

In a further step 104, in accordance with the present disclosure the method 100 involves subjecting portions of the compounded PCR blend to testing methods to evaluate the efficacy of additives in resisting oxidation after utilization. In a specific embodiment, the efficacy of additives in resisting oxidation after utilization is evaluated using Oxidative Induction Time (OIT) testing method. The OIT testing method is apparent to those having ordinary skill in the art, is conducted to assess the efficiency and stability of additives post-utilization, measuring their ability to prevent oxidation. The OIT testing methodology in accordance with the present disclosure assesses the efficiency of additives in resisting oxidation, providing critical insights into the oxidative stability of the PCR blend. It helps in evaluating how well the additives maintain their effectiveness in preventing oxidation.

The method 100 as disclosed further comprises the step 105, involving assessment of stability and efficiency of the quantified additives in recycled materials after utilization. In a specific embodiment of the present disclosure, the analysis of the additive content serves as a predictive tool for determining the suitability of PCR blends for specific applications after a certain number of re-utilization cycles. Specifcally, in step 106, the cumulative effect of utilization on the stability and performance of additive in the PCR blend is assessed.

The analytical techniques and testing method employed in accordance with the present disclosure, including HPLC technique and OIT testing, enabled a nuanced understanding of the additive behavior, facilitating the optimization of recycling processes by adding optimised additive in PCR blend and the development of sustainable polymer materials with enhanced performance properties.

In accordance with the method 100 of the present disclosure, the correlation is determined by assessing changes in the efficiency of the additives, as measured by OIT, across multiple re-utilization cycles. The disclosed method 100 enables establishment of a correlation through statistical analysis, regression analysis, or machine learning algorithms applied to the data obtained from HPLC technique step 103 and OIT testing step 104. Further, in accordance with the method 100, the efficiency of the additive content in the PCR blend is determined by the consecutive cycles of compounding the PCR blends and subjecting them to the analysis steps 105 and 106.

The method 100 as disclosed enables a clear representation of additive concentrations and variations in the PCR blends, based on the results obtained from HPLC and OIT testing in graphical or tabular formats. Further, the HPLC analysis is integrated into a feedback control system, enabling real-time adjustments to the compounding process based on observed additive concentrations. In an embodiment, the results obtained from the HPLC and OIT testing are presented in a clear and comprehensive manner. Graphical or tabular formats are employed to provide a visual representation of additive concentrations and variations in the PCR blends. This visual representation has aided the inventors in interpreting and communicating the analytical findings.

In accordance with the method 100 of the present disclsoure, the quantified additive content and/or additives degradation products within the PCR blend as identified, further enables end user to correlate the efficiency of the individual additive content in PCR with number of times of re-utilization of the PCR blend. The correlation thus established between the additive content and the number of times of re-utilization of the PCR blend is utilized to provide recommendations for adjusting recycling practices or modifying additive formulations for enhanced performance of the polymer material.

For example, in a benefitting embodiment, the correlation directly provides insights into how the additive concentrations and effectiveness evolve over successive or multiple recycling cycles. In a most advantageous embodiment, the correlation is utilized to optimize the composition or concentration of the additives or adjust processing parameters for improved performance of the PCR polymer over multiple re-utilization cycles. Furthermore, advantageously the correlation exhibited between the efficiency of the additive content in PCR with number of times of re-utilization of the PCR blend, is utilized to predict the longevity and effectiveness of additives in PCR blends, providing insights for sustainable material use.

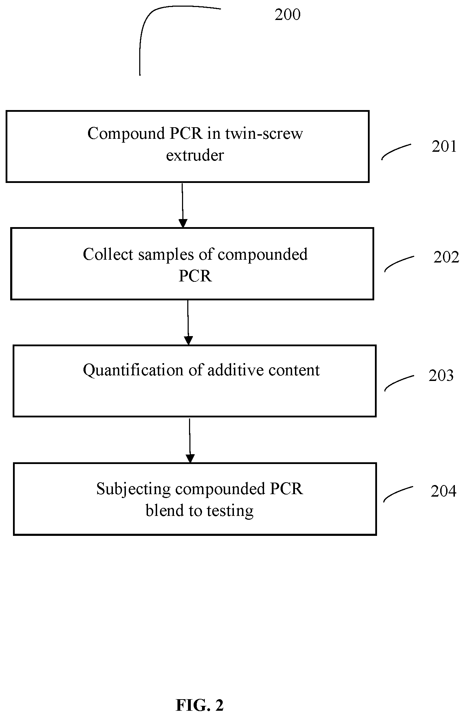

The present disclosure also provides a method 200 for determining additive content in Post-Consumer Recycled (PCR) blends as discloed in FIG. 2. The method comprises step 201, compounding an obtained PCR polymers in a twin-screw extruder. In accordance with the present disclosure the PCR polymer compounded in the twin-screw extruder is obtained from plastic waste sources including but not limited to recycled polypropylene, recycled polyethylene, preferably recycled high density polyethylene(HDPE), recycled low density polyethylene(LDPE), recycled linear low density polyethylene(LLDPE), recycled medium density polyethylene(MDPE), or a mixture thereof. In a preferred embodiment, the PCR polymer compounded in the twin-screw extruder is recycled polyethylene.

In another embodiment, the obtained polymer is recycled polystyrene. In yet another embodiment, the polymer is poly(dimethylsiloxane). In another embodiment, the obtained polymer is a polypropylene homopolymer or a primarily polypropylene copolymer. In another embodiment, the obtained polymer is a polyethylene homopolymer or a primarily polyethylene copolymer.

The PCR polymer is subjected to compounding in the twin-screw extruder under controlled temperature and pressure conditions. For example at temperatures greater than 150 °C, preferably ranging between 150 °C to 500 °C, and pressure greater than 70 MPa, preferably ranging between 70 MPa to 700 MPa. In some embodiments of the present disclosure, the PCR polymer is also compounded as a mixture including compatibilizers, pigments, dyes, process aides, additives, and/or a mixture thereof in the twin-screw extruder under controller temperature and pressure to facilitate homogeneous mixing and distribution of components.

Non-limiting examples of pigments include organic pigments, such as copper phthalocyanine, inorganic pigments, such as titanium dioxide, and other pigments that may be apparent to those having ordinary skill in the art. A non-limiting example of an organic dye includes Basic Yellow 51. Non-limiting examples of process aides are antistatic agents, such as glycerol monostearate and slip-promoting agents, such as erucamide. Compatibilizers include for example, but not limited to maleic anhydride grafted polymers, polyethylene-grafted maleic anhydride, polypropylene-grafted maleic anhydride, silane grafted polyethylene, acrylic acid grafted polyethylene, glycidyl acrylate grafted polyethylene, glycidyl methacrylate grafted polypropylene, hydroxyethyl methacrylate grafted polyethylene and aminoethyl methacrylate grafted polypropylene.

The method further involves step 202, where the samples of the compounded PCR blend are collected at predetermined intervals during the extrusion process. The intervals may be defined based on specific time durations or the completion of defined extruder revolutions, at least 2, preferably between 5 and 10, ensuring a comprehensive representation of the blends composition.

In accordance with the present disclosure, the method 200 involves step 203, quantifying concentrations of individual additives and/or additives degradation products present in the collected PCR blend samples using analytical techniques. The quantification of the concentration of the additives and/or additives degradation products is performed using well known analytical techniques such as High-Performance Liquid Chromatography (HPLC), Gel Permeation Chromatography (GPC), Nuclear Magnetic Resonance (NMR) Spectroscopy, Infrared Spectroscopy (IR), or Mass Spectroscopy (MS). In a preferred embodiment in accordance with the present disclosure, the analytical technique is High-Performance Liquid Chromatography.

In accordance with an embodiment of the present disclosure, collected samples in step 202 undergo HPLC analysis step 203 to quantify the concentrations of individual additives and/or additives degradation products present in the PCR blend. The HPLC technique aids in separating and quantifying individual additives and/or additives degradation products within the PCR blend, ensuring precise measurement of additive concentrations. The HPLC analysis includes a separation step that isolates individual additives and/or additives degradation products present in the PCR blend, thereby enabling an accurate quantification. The HPLC analysis is conducted using calibration standards, internal standards, or both, to ensure accuracy and reproducibility of additive concentration measurements.

In an embodiment of the present dislclosure the HPLC analysis results identifying the individual additives content and/or additives degradation products within the PCR blend is coupled with data analytics techniques, statistical analysis, or machine learning algorithms to extract patterns, trends, and / or correlations which further help in assessing the additive behaviour during re-utilization cycles.

Non-limiting examples of additives include antioxidants, stabilizers, or colorants. In a specific embodiment the additive is selected from the group comprising antioxidants, stabilizers, or colorants, including BHT (Butylated Hydroxytoluene), BHA (Butylated Hydroxyanisole), Tris(2,4-di-tert-butylphenyl)phosphite (TNPP), Triphenyl phosphite, Dilauryl thiodipropionate (DLTDP), Dimyristyl thiodipropionate (DMTDP), Calcium stearate, Zinc stearate, Titanium dioxide, Iron oxide pigments, Fatty acid esters and / or Calcium stearate. In a preferred embodiment the additive is selected from the group comprising antioxidants, stabilizers, Calcium stearate, Zinc stearate.

In a further step 204, in accordance with the present disclosure the method 200 involves subjecting portions of the compounded PCR blend to testing methods to evaluate the efficacy of additives in resisting oxidation after utilization. In a specific embodiment, the efficacy of additives in resisting oxidation after utilization is evaluated using Oxidative Induction Time (OIT) testing method. The OIT testing method is apparent to those having ordinary skill in the art, is conducted to assess the efficiency and stability of additives post-utilization, measuring their ability to prevent oxidation.

In some embodiments, the steps 201 to 204 in the same sequence are repeated to analyse the additive concentration for various multiple recycle cycles of the PCR blend. The method 200 as disclosed enables a clear representation of additive concentrations and variations in the PCR blends, based on the results obtained from HPLC and OIT testing in graphical or tabular formats. Further, the HPLC analysis is integrated into a feedback control system, enabling real-time adjustments to the compounding process based on observed additive concentrations. In an embodiment, the results obtained from the HPLC and OIT testing are presented in a clear and comprehensive manner. Graphical or tabular formats are employed to provide a visual representation of additive concentrations and variations in the PCR blends. This visual representation has aided the inventors in interpreting and communicating the analytical findings.

The present disclosure further provides a method 300 for producing a molded article. The method in accordance with the present disclosure includes, providing an obtained recycled polymer feedstock 301 and subjecting it to melt-compounding conditions sufficient to form an article. The compounding conditions are implemented in the compounding zone of a twin screw extruder 302 or mixer and are tailored for mixtures. For example in some embodiments, the obtained recycled polymer is compounded along with specific polyolefins, chain extenders or peroxides, additional additives and optionally a virgin polymer. The addition of additives under controlled temperature of 170 to 240°C, pressure, and shear force conditions are implemented in the extruder or mixer sufficient to provide homogeneous mixing of the recycled polymer and the additives to produce a substantially homogeneous polymer blend. In another embodiment the obtained recycled polymer is compounded without any addition of additives.

The method 300 as disclosed in step 302, the compounding conditions will be such that the specific energy from the compounder from shear and/or added heat are sufficient to melt the recycled polymer composition and homogenize them. In some embodiments, compounding conditions comprise a temperature in the compounding zone of less than or equal to 300°C, less than or equal to 250°C. In some embodiments, where the recycled polymer comprises a polyolefin, temperatures in the compounding zone can be in the range of from 170°C to 240°C. In a further step 303, the recycled polymer can be pelletized and obtained in the form of recycled polymer pellets.

In accordance with the method 300 of the present disclosure, the recycled polymer pellets are further blow molded 304 to produce a recycled polymer product. The recycled polymer pellets are thoroughly cleaned and processed to remove contaminants and impurities, as this is crucial to ensure the final product's quality and performance. The cleaned recycled polymer pellets are then melted using heat. This is performed in an extruder, where the pellets are subjected to high temperatures to become a molten, viscous material.

The molten polymer is then extruded through a hollow tube of plastic or a parison and placed within a mold cavity. Once the parison is in the mold, pressurized air is injected into it which causes the molten plastic to expand and take the shape of the mold. The mold is further cooled to solidify the plastic in the desired article form. The molded product or molded article is further allowed to cool within the mold. In accordance with the present dislcosure, the cooling time is crucial for ensuring the product retains its shape and structural integrity. After solidification and cooling, the mold is opened, and the formed product or article is ejected. In some embodiments of the present discloure this can be an automated or manual process.

In one embodiment of the present disclosure the additives is introduced after the addition of the recycled polymer feedstock. In some embodiments, the additive is introduced simultaneously during the addition of the recycled polymer feedstock. In another embodiment, the additve is introduced as a masterbatch with the recycled polymer feedstock. The additive introduced in the feedstock is optimised based on the efficiency of the additive content in PCR with number of times of re-utilization of the PCR blend.

In every embodiment of the present disclosure, the addition of optimised content of additive to the recycled polymer resulted in longevity and effectiveness of additives in PCR blends and an article produced thereby. For example, OIT, thermal stability, mechanical properties.

The present disclosure also provides a molded article which is produced in accordance with the method 300. The recycled polymer article thus produced which is incorportaed with an optimised amount of additive, based on the correlation the evolving or deteriorating efficiency of the additive in the re-utilization of the PCR blends. Non-limiting examples of the article thus produced can be pipe, bottles, containers, bags, automotive components for both interiror and exterior applictaions, sustainable building materials such as plastic lumber, folders, binders, etc.

For the sake of brevity, only certain ranges are explicitly disclosed herein. However, in addition to recited ranges, any lower limit may be combined with any upper limit to recite a range not explicitly recited, as well as, ranges from any lower limit may be combined with any other lower limit to recite a range not explicitly recited, in the same way, ranges from any upper limit may be combined with any other upper limit to recite a range not explicitly recited. Additionally, within a range includes every point or individual value between its end points even though not explicitly recited. Thus, every point or individual value may serve as its own lower or upper limit combined with any other point or individual value or any other lower or upper limit, to recite a range not explicitly recited.

All documents and references cited herein, including testing procedures, publications, patents, journal articles, etc., are herein fully incorporated by reference for all jurisdictions in which such incorporation is permitted and to the extent such disclosure is consistent with the description of the present disclosure.

Although the present disclosure and its advantages have been described in detail, it should be understood that various changes, substitutions and alterations can be made herein without departing from the spirit and scope of the disclosure as defined by the appended claims. Moreover, the scope of the present application is not intended to be limited to the particular embodiments of the processes, machines, means, methods, and/or steps described in the specification. As one of the ordinary skill in the art will readily appreciate from the disclosure of the present disclosure, processes, machines, means, methods, and/or steps, presently existing or later to be developed that perform substantially the same function or achieve substantially the same result as the corresponding embodiments described herein, may be utilized according to the present disclosure. Accordingly, the appended claims are intended to include within their scope such processes, machines, means, methods, and/or steps.

## Claims

1. A method for determining re-utilization efficiency of an additive content in Post-Consumer Recycled (PCR) blends, the method comprising the steps of:
a. compounding an obtained Post-Consumer Recycled polymers in a twin-screw extruder, under controlled temperature and pressure conditions;
b. collecting samples of the compounded PCR blend at predetermined intervals during the extrusion process;
c. quantifying concentrations of individual additives and/or additives degradation products present in the collected PCR blend samples using analytical techniques, thereby determining the additive content and/or additive degradation product content post-utilization;
d. subjecting portions of the compounded PCR blend to testing methods to evaluate the efficacy of additives in resisting oxidation after utilization;
e. assessing stability and efficiency of additives in recycled materials after utilization; and,
f. assessing cumulative effect of utilization on the stability and performance of additives in the PCR blends;
**characterized in that** the quantified additive content, enables end user to correlate the efficiency of the additive content in PCR with number of times of re-utilization of the PCR blend.

2. The method according to claim 1, wherein the efficiency of the additive content in the PCR blend is determined by the consecutive cycles of compounding the PCR blends and subjecting the PCR blends to the analysis steps e to f.

3. The method according to any of the preceding claims, wherein the quantified additive content in step c, is determined by using the techniques including High-Performance Liquid Chromatography (HPLC), Gel Permeation Chromatography (GPC), Nuclear Magnetic Resonance (NMR) Spectroscopy, Infrared Spectroscopy (IR), or Mass Spectroscopy (MS), preferably HPLC.

4. The method according to any of the preceding claims, wherein the efficacy of additives in resisting oxidation after utilization is evaluated using Oxidative Induction Time (OIT) testing method.

5. The method according to any of the preceding claims, wherein the correlation is utilized to optimize the composition of additives or adjust processing parameters for improved performance over multiple re-utilization cycles.

6. The method according to any of the preceding claims, wherein the additive is selected from the group comprising antioxidants, stabilizers, or colorants, including BHT (Butylated Hydroxytoluene), BHA (Butylated Hydroxyanisole), Tris(2,4-di-tert-butylphenyl)phosphite (TNPP), Triphenyl phosphite, Dilauryl thiodipropionate (DLTDP), Dimyristyl thiodipropionate (DMTDP), Calcium stearate, Zinc stearate, Titanium dioxide, Iron oxide pigments, Fatty acid esters and / or Calcium stearate.

7. The method according to any of the preceding claims, wherein the HPLC analysis is coupled with data analytics techniques, statistical analysis, or machine learning algorithms to extract patterns, trends, and / or correlations in additive behaviour during re-utilization cycles.

8. The method according to any of the preceding claims, wherein the correlation is utilized to predict the longevity and effectiveness of additives in PCR blends, providing insights for sustainable material use.

9. A method for determining additive content in Post-Consumer Recycled (PCR) blends, the method comprising the steps of:
a. compounding an obtained Post-Consumer Recycled polymers in a twin-screw extruder, under controlled temperature and pressure conditions;
b. collecting samples of the compounded PCR blend at predetermined intervals during the extrusion process;
c. quantifying concentrations of individual additives and/or additives degradation products present in the collected PCR blend samples using analytical techniques, thereby determining the additive content post-utilization; and
d. subjecting portions of the compounded PCR blend to testing methods to evaluate the efficacy of additives in resisting oxidation after utilization.

10. The method according to claim 9, wherein the Post-Consumer Recycled polymers are obtained from plastic waste sources, including but not limited to recycled polypropylene, recycled polyethylene, preferably recycled high density polyethylene(HDPE), recycled low density polyethylene(LDPE), recycled linear low density polyethylene(LLDPE), recycled medium density polyethylene(MDPE), or a mixture thereof.

11. A method for producing a molded article comprising the steps of:
a) providing a recycled polymer feedstock;
b) extruding the recycled polymer feedstock at a temperature of 170 to 240°C;
c) obtaining recycled polymer pellets;
d) blow molding the recycled polymer pellets to form a recycled polymer product;
e) adding the additive in step (a) determined by correlating the efficiency of the additive content in PCR with number of times of re-utilization of the PCR blend;
wherein the addition of optimized content of additive to the recycled polymer results in longevity and effectiveness of additives in PCR blends.

12. A molded article produced according to claim 11.

13. The molded articled according to claim 12, wherein the article is selected from the group comprising but not limited to pipe, bottles and containers.
